# EUROPÄISCHE PATENTSCHRIFT

(11) **EP 2 029 039 B1**
(45) Veröffentlichungstag und Bekanntmachung des Hinweises auf die Patenterteilung: **15.12.2010**
(21) Anmeldenummer: 07765344.2
(22) Anmeldetag: 08.06.2007
(51) Int. Cl.: A61B 18/14, A61B 18/00

(54) **VORRICHTUNG ZUM SCHNEIDEN UND KOAGULIEREN VON GEWEBE**
DEVICE FOR CUTTING AND COAGULATION OF TISSUE
DISPOSITIF DE DÉCOUPAGE ET DE COAGULATION DE TISSUS

(30) Priorität: 08.06.2006 DE 102006027150
(43) Veröffentlichungstag der Anmeldung: 04.03.2009
(73) Patentinhaber: Celon AG Medical Instruments, 14513 Teltow (DE)
(72) Erfinder: DESINGER, Kai, 14195 Berlin (DE); ROGGAN, André, 12163 Berlin (DE); STEIN, Thomas, 14513 Teltow (DE); KÜHNE, Wolfgang, 16567 Schönfliess (DE)
(74) Vertreter: Eisenführ, Speiser & Partner
(86) Internationale Anmeldenummer: PCT/EP2007/055669
(87) Internationale Veröffentlichungsnummer: WO 2007/141337

(56) Entgegenhaltungen:
- EP-A- 1 527 743
- WO-A-2004/078051
- DE-A1- 4 017 626
- US-A- 2 004 559
- US-A- 4 492 231
- US-B1- 6 926 717

## Beschreibung

Die Erfindung betrifft ein Instrument für das Trennen oder Entfernen von Körpergewebe mittels Elektrotomie und das Veröden von Körpergewebe bzw. das Stillen von Blutungen mittels Elektrokoagulation.

Die Nutzung elektrochirurgischer Verfahren zur Gewebetrennung oder Gewebeentfernung (Elektrotomie) ist bereits seit Jahrzehnten ein Routineverfahren in der Chirurgie. Solche Verfahren bieten den Vorteil, dass das Gewebe in Form eines sogenannten Schmelzschnittes getrennt wird, bei dem von der Schneidelektrode eine Funkenentladung ausgeht, die das Gewebe in unmittelbarer Umgebung der Schneidelektrode zur Verdampfung bringt und kleine Kapillargefäße umgehend verschließt, sodass ein fast blutfreies Gewebeschneiden möglich ist. Dieses Verfahren wird in allen chirurgischen und anderen medizinischen Disziplinen zur Gewebetrennung oder Gewebeentfernung eingesetzt.

Das elektrochirurgische Veröden von Gewebe bzw. das Stillen von Blutungen mittels Hochfrequenzstrom ist ebenfalls ein in der Chirurgie und anderen medizinischen Disziplinen etabliertes Verfahren. Die hierzu verwendeten Instrumente sind als Greifinstrumente wie z. B. Pinzetten oder Zangen ausgeführt. Am distalen Ende der Schenkel des Greifinstrumentes sind großflächige Elektroden angebracht, die auf verschiedenem (bipolar) oder gleichem (monopolar) Potential liegen. Sowohl bei der monopolaren als auch bei der bipolaren Anwendung des Greifinstrumentes befindet sich das zu verödende Gewebestück bzw. das Ende des zu verschließende Blutgefäßes zwischen den Elektroden am distalen Ende des Greifinstrumentes. Bei der monopolaren Technik fließt der Hochfrequenzstrom zwischen diesen Elektroden und einer an anderer Stelle am Patienten befestigten Rückleitelektrode. Bei der bipolaren Technik fließt der Hochfrequenzstrom zwischen den beiden Elektroden am distalen Ende der Schenkel des Greifinstrumentes.

Das Schneiden mittels Draht- bzw. Lanzettenelektroden, die sich am Ende eines Handgriffes befinden und das Koagulieren mit Greifinstrumenten sind die am weitesten verbreiteten hochfrequenzchirurgischen Techniken. Beim derzeitigen Stand der Technik ist es so, dass die Schneidelektrode und das Koagulationsgreifinstrument zwei verschiedene Instrumente sind, die während eines operativen Eingriffes oft gewechselt werden müssen bzw. von zwei Operateuren bedient werden.

Bei Operationen im offenen Bauchraum ist dies kein größeres Problem, aber bei minimal-invasiven Eingriffen wie der Laparoskopie oder bei Operationen im Mund- oder Rachenraum stellt dies ein größeres Problem dar. Bei der Laparoskopie muss für jedes Instrument ein Trokar gesetzt werden. Will man auf zusätzliche Trokare verzichten, müssen die Instrumente während des Eingriffes oft gewechselt werden, was sich bei der Laparoskopie als zeitaufwendig darstellt.

Eingriffe im Mund- und Rachenraum werden wegen des zur Verfügung stehenden geringen Einblickwinkels in diese natürliche Körperöffnung nur von einem Operateur durchgeführt. Dies bedeutet, dass beim Übergang vom Schneiden auf das Koagulieren beim derzeitigen Stand der Technik ein Instrumentenwechsel notwendig ist. Oft ist es so, dass bei der Durchführung eines Hochfrequenzschnittes und beim Durchtrennen eines größeren Blutgefäßes die blutstillende Eigenschaft des koagulierten Schnittsaumes nicht ausreicht und zusätzlich mit einem bipolaren oder monopolaren Greifinstrument die Blutung gestoppt werden muss. Bei einem solchen Fall ist ein sehr schneller Wechsel vom schneidenden auf das koagulierende Instrument sehr wichtig.

Aus DE 40 17 626 A1 ist eine Kombination aus einem schneidenden und einem koagulierenden instrument bekannt.

Der Erfindung liegt die Aufgabe zugrunde, eine Applikationsvorrichtung für die Elektrotomie zu schaffen, die die Nachteile des Standes der Technik möglichst vermeidet.

Erfindungsgemäß besteht die Lösung dieser Aufgabe in einem elektrochirurgischen Instrument, das als Greifinstrument ausgeführt ist und die Funktionen Schneiden, Koagulieren und Greifen in einem Instrument vereint.

Dazu ist das Instrument als ein Greifinstrument mit zwei relativ zueinander beweglichen Greifarmen ausgeführt, die jeweils an ihrem distalen Ende je wenigstens zwei Elektroden, nämlich wenigstens eine Koagulationselektrode und wenigstens eine Schneideelektrode aufweisen.

Eine jeweilige Schneidelektrode ist als an der Außenseite eines jeweiligen distalen Endes eines Greifarmes angeordneter Draht ausgebildet, der somit als Schneiddraht wirkt. Dadurch kann das Greifinstrument mit geschlossenen Greifarmen in für den Arzt gewohnter Weise wie ein mono- oder bipolares Schneidinstrument herkömmlicher Bauart verwendet werden.

Die Koagulationselektroden sind vorzugsweise als leitende Oberflächen auf einander zugewandten Innenflächen an den distalen Enden der Greifarme ausgeführt. Eine mechanische Wegbegrenzung für die Bewegung der Greifarme verhindert vorzugsweise eine gegenseitige Berührung der die Koagulationselektroden bildenden leitenden Oberflächen und somit einen ungewollten Kurzschluss. Die mechanische Wegbegrenzung ist vorzugsweise von einem in Richtung des jeweils anderen Greifarms gerichteten Vorsprung auf einer Innenfläche eines Greifarmes gebildet.

Vorzugsweise weist wenigstens einer der Greifarme einen Saugkanal mit einer Ansaugöffnung im Bereich des distalen Endes des Greifarmes auf, der an ein Absaugsystem anzuschließen und ausgebildet ist, Flüssigkeit im Bereich des distalen Endes des Greifarmes abzusaugen. Der Saugkanal dient dazu, Blut oder andere Körperflüssigkeit abzusaugen, die sich am Eingriffsort sammelt.

Außerdem weist vorzugsweise wenigstens einer der Greifarme einen Spülkanal auf, der in einem offenen Ende in der Nähe des distalen Endes des Greifarmes mündet und derart ausgebildet ist, dass er mit seinem proximalen Ende an eine Quelle für Spülflüssigkeit anzuschließen ist und Spülflüssigkeit über seine offene Mündung am distalen Ende austreten kann. Mit Hilfe eines derartigen Spülkanals kann der Eingriffsort mit Spülflüssigkeit gespült werden.

Die Schneidelektroden sind vorzugsweise einander parallelgeschaltet. Außerdem weist die Applikationsvorrichtung vorzugsweise eine separate Neutralelektrode auf, die mit einem anderen Pol eines Schneidstromgenerators zu verbinden ist, als die beiden Schneidelektroden. Auf diese Weise arbeitet die Applikationsvorrichtung als monopolares Schneidinstrument.

Die Erfindung wird nachfolgend anhand von Ausführungsbeispielen und der dazugehörigen Zeichnungen näher erläutert. Es zeigen:
- Figur 1:: ein erfindungsgemäßes elektrochirurgisches Instrument ähnlich einer bipolaren oder monopolaren Pinzette oder Zange, an dessen Innenseiten der distalen Enden der Schenkel sich die großflächigen Elektroden zur Gewebe- koagulation befinden (Kontaktfläche 1 und 2) und an des- sen Außenseiten der distalen Enden der Schenkel Schneiddrähte (Kontaktdraht 1 und 2) angebracht sind;
- Figur 2:: eine beispielhafte, vergrößerte schematische Schnittdar- stellung einer Instrumentenführung bei der elektrochirurgi- schen Trennung (Elektrotomie) von organischem Gewebe mittels der außen am Schenkel angebrachten Schneiddräh- te (Kontaktdraht 1 und 2);
- Figur 3::: eine beispielhafte, vergrößerte schematische Schnittdar- stellung einer Instrumentenführung bei der Hochfrequenz- Koagulation (Elektrokoagulation) von organischem Gewebe mittels der an den innen an den Schenkeln befindlichen Kontaktflächen (Kontaktfläche 1 und 2);
- Figuren 4a und 4b:: eine beispielhafte, vergrößerte schematische Schnittdar- stellung einer Ausführungsvariante mit einer Kurzschlusssi- cherung, die dafür sorgt, dass auch bei geschlossenen Schenkeln ein minimaler Spalt zwischen den Kontaktflä- chen (Kontaktfläche 1 und 2) vorhanden bleibt und somit verhindert, dass sie sich berühren können;
- Figuren 5a, 5b und 5c:: beispielhafte, vergrößerte schematische Schnittdarstel- lungen von verschiedenen Ausführungsvarianten mit unter- schiedlich aufgebrachten Kontaktflächen oder integrierten Kontaktflächen oder eingelassenen Kontaktflächen;
- Figur 6:: eine Ausführungsvariante mit einem Absaugkanal, der im unmittelbaren Arbeitsbereich des Instrumentes Flüssigkei- ten, wie z. B. Blut, absaugt und abtransportiert;
- Figur 7:: eine Ausführungsvariante mit einem Spülkanal, durch den das Gewebe im Arbeitsbereich mit Flüssigkeiten, wie z. B. sterile physiologische Kochsalzlösung befeuchtet werden kann, um eine Austrocknung des Gewebes zu verhindern.
- Figur 8:: eine Ausführungsvariante mit austauschbaren Schneid- und Koagulationslektroden;
- Figuren 9a bis e:: einen Hochfrequenzchirurgiegenerator (Fig. 9a) und ver- schiedene daran anzuschließende Applikationsvorrichtun- gen einschließlich der Verdrahtung der jeweiligen Elektro- den;
- Figur 10:: ein Anschlusskabel zum Anschluss des erfindungsgemä- ßen elektrochirurgischen Instrumentes an den Hochfre- quenzgenerator gemäß Fig. 9; und
- Figur 11:: ein Anschlusskabel zum Anschluss einer Neutralelektrode an den Hochfrequenzgenerator gemäß Fig. 9.

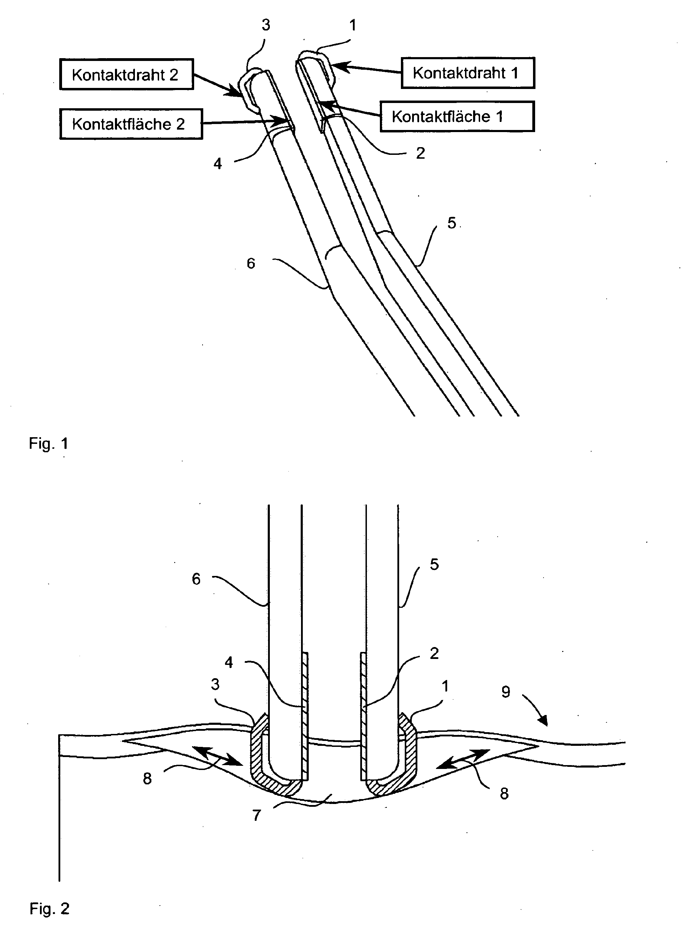

Figur 1 zeigt ein erfindungsgemäßes elektrochirurgisches Instrument, dass ähnlich einer bipolaren oder monopolaren Pinzette oder Zange aufgebaut ist, und an Innenseiten von distalen Enden von Greifarme bildenden Schenkeln 5 und 6 großflächige Elektroden 2 und 4 zur Gewebekoagulation aufweist, die in Figur 1 auch als Kontaktflächen 1 und 2 bezeichnet sind. An Außenseiten der distalen Enden der Schenkel 5 und 6 sind Schneiddrähte 1, 3 angebracht, die in Figur 1 auch als Kontaktdraht 1 und 2 bezeichnet sind.

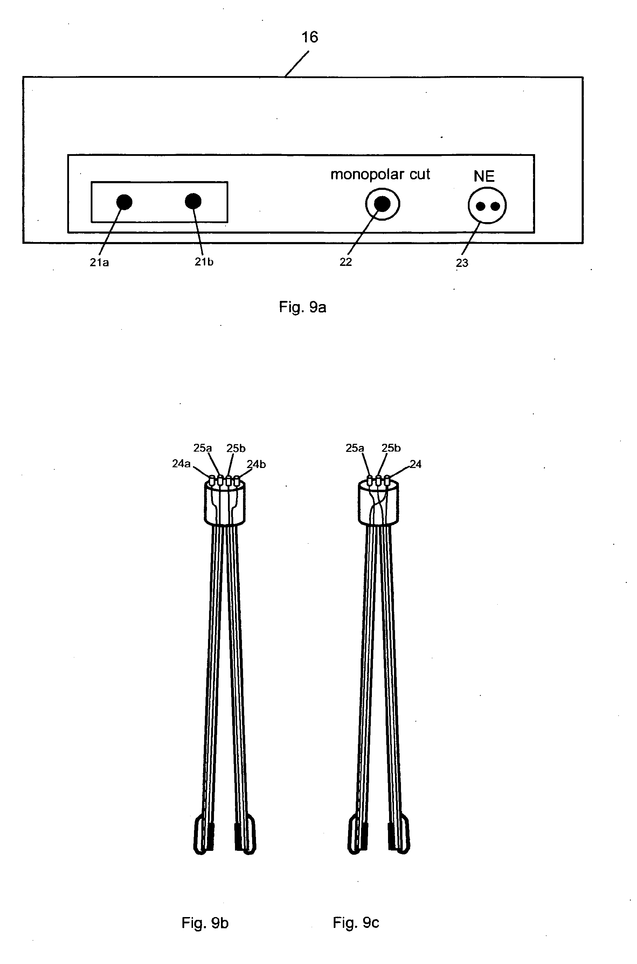

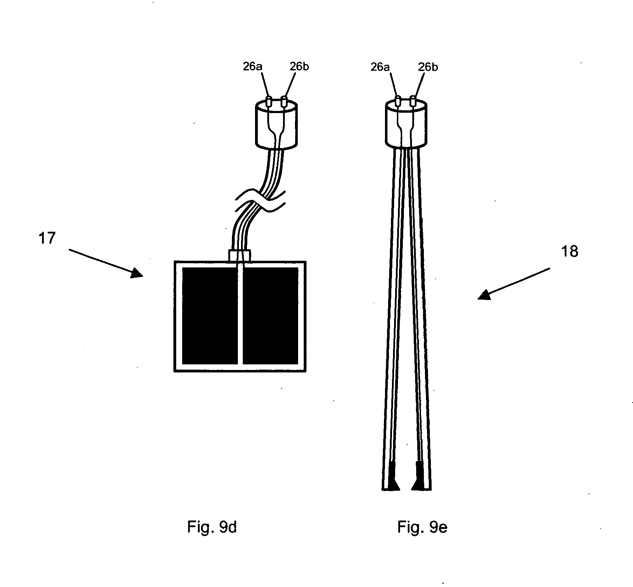

Auf diese Weise ergibt sich ein chirurgisches Instrument mit insgesamt vier Elektroden (zwei kleinflächige Drahtelektroden 1 und 3 und zwei großflächige Elektroden 2 und 4). Für diese vier Elektroden 1 bis 4 sind folgende Verschaltungstechniken/Strompfade denkbar:, die in Figuren 9 a bis e auch bildlich dargestellt sind:

### Schneidvorgang:

S1) Kontaktdraht 1 zu Kontaktdraht 2
S2) Kontaktdraht 1 zu Kontaktfläche 1
S3) Kontaktdraht 1 zu Kontaktfläche 2
S4) Kontaktdraht 1 zu Kontaktfläche 1 und 2
S5) Kontaktdraht 2 zu Kontaktfläche 2
S6) Kontaktdraht 2 zu Kontaktfläche 1
S7) Kontaktdraht 2 zu Kontaktfläche 2 und 1
S8) Kontaktdraht 1 und 2 zu Kontaktfläche 1
S9) Kontaktdraht 1 und 2 zu Kontaktfläche 2
S10) Kontaktdraht 1 und 2 zu Kontaktfläche 1 und 2
S11) Kontaktdraht 1 zu separater Rückleitelektrode
S12) Kontaktdraht 2 zu separater Rückleitelektrode
S13) Kontaktdraht 1 und 2 zu separater Rückleitelektrode

### Koagulationsvorgang:

K1) Kontaktfläche 1 zu Kontaktfläche 2
K2) Kontaktfläche 1 und 2 zu separater Rückleitelektrode

Das elektrochirurgische Instrument (auch Applikationsvorrichtung genannt) bildet ein Kombinationsinstrument, das mit einem Anschluss versehen ist, der eine elektrische Verbindung mit mehreren Ausgängen eines Hochfrequenzchirurgiegenerators 16 (Electro Surgical Unit; siehe Figur 9a) ermöglicht. Der Hochfrequenzchirurgiegenerator 16 besitzt entweder mindestens einen Schneidausgang 22 und einen Koagulationsausgang21a und 21 b, oder einen Kombinationsausgang, der sowohl eine Schneid- als auch eine Koagulationsspannung liefert. Bei einer Verbindung des Kombinationsinstrumentes mit einem konventionellen Hochfrequenzchirurgiegenerator, der über Ausgänge für monopolares Schneiden, monopolares Koagulieren, bipolares Schneiden und bipolares Koagulieren verfügt, sind unter anderem folgende Anschlussvarianten denkbar:
Ausgang für monopolares Schneiden für: S11 bis S13
Ausgang für monopolare Koagulation für: K2
Ausgang für bipolares Schneiden für: S1 bis S10
Ausgang für bipolare Koagulation für: K1

Der Hochfrequenzchirurgiegenerator 16 aus Figur 9a besitzt einen Anschluss 21 a und 21 b für bipolares Koagulieren (bipolar coag) und einen Anschluss 22 für monopolares Schneiden (monopolar cut) und erlaubt daher die Schaltungsvarianten K1 und S11 bis S13, wenn er mit einem chirurgischen Instrument betrieben wird, wie es in Figur 9b dargestellt ist. Bei jenem chirurgischen Instrument aus Figur 9b sind am proximalen Ende des Instrumentes insgesamt vier Anschlusskontakte 24a, 24b, 25a und 25b vorgesehen, die es erlauben, jede der Elektroden 1 bis 4 einzeln mit einem Hochfrequenzchirurgiegenerator zu verbinden. Der Hochfrequenzchirurgiegenerator aus Figur 9a in Verbindung mit einem chirurgischen Instrument wie in Figur 9c abgebildet erlaubt die Schaltungsvarianten K1 und S13. Bei dem chirurgischen Instrument aus Figur 9c sind beide Schneiddrähte 1 und 3 mit einem gemeinsamen Anschlusskontakt 24 am proximalen Ende des Instrumentes verbunden und daher parallelgeschaltet.

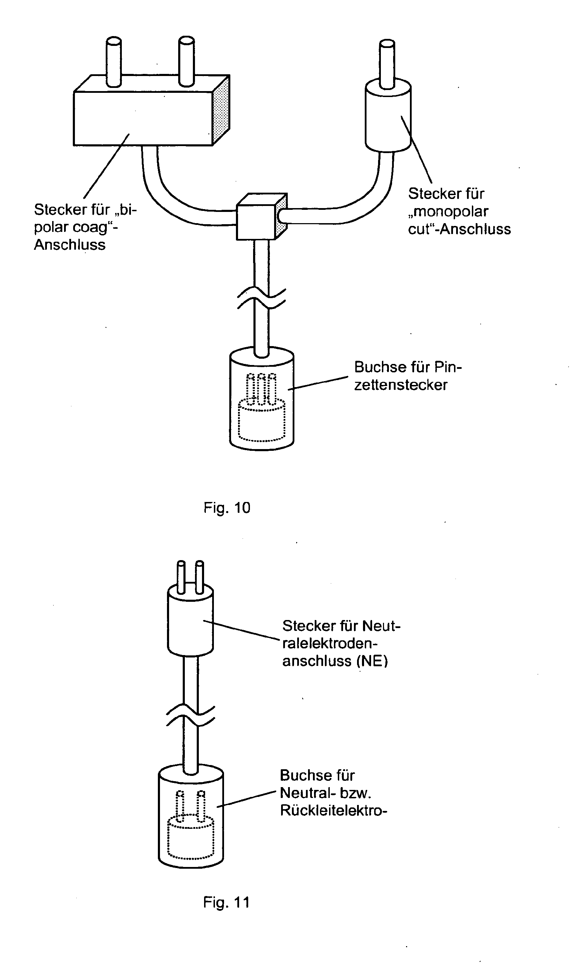

Außerdem besitzt der Hochfrequenzchirurgiegenerator 16 einen Anschluss 23 zum Anschluss einer Neutralelektrode (siehe Figuren 9d oder 9e) bzw. eines Anschlusskabels für eine derartige Neutralelektrode (siehe Figur 11).

Figuren 9d und 9e zeigen jeweils insbesondere für das monopolare Gewebeschneiden geeignete Neutralelektroden, und zwar zeigt Figur 9d eine Flächenelektrode 17 als Neutralelektrode und Figur 9e eine Neutralelektrode in Form einer Pinzette 18.

Figur 2 zeigt einen beispielhaften Schneidprozess, in dem durch das Führen 8, der am Instrument außen liegenden Schneiddrähte 1 und 3 mit leichter Berührung des Gewebes 9 entlang der Schneidkante 7 der Schneidvorgang stattfindet.

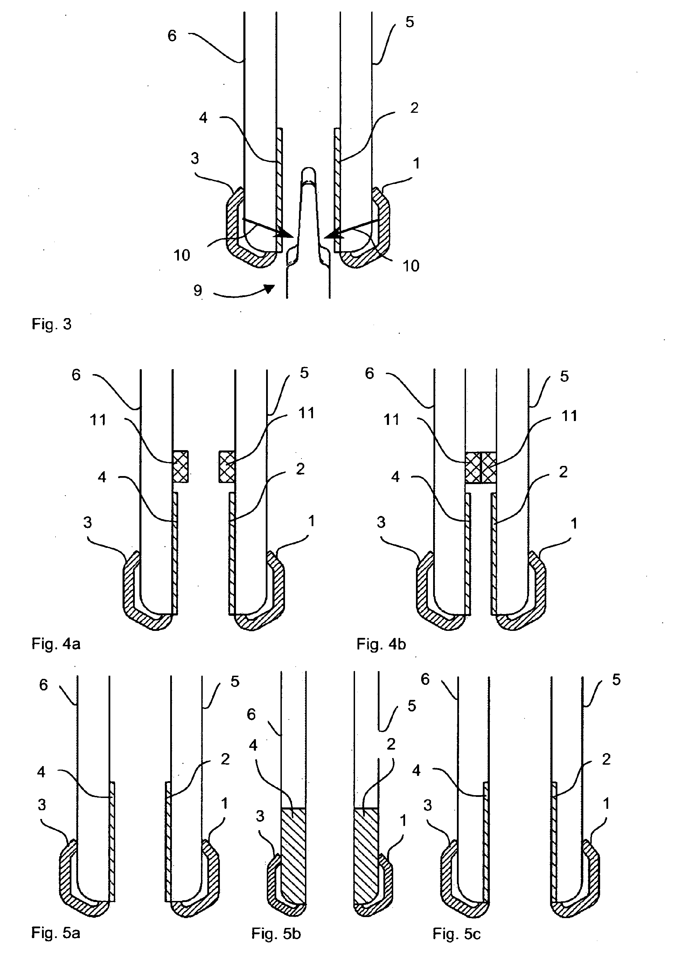

Figur 3 zeigt einen beispielhaften Koagulationsprozess, in dem das zu koagulierende Gewebe 9 zwischen den am Instrument innen liegenden Kontaktflächen 2 und 4 mit leichtem Druck in Richtung 10 gegriffen wird.

Bei der bipolaren Elektrokoagulation mit einem zangenähnlichen Werkzeug besteht grundsätzlich dann die Gefahr eines Kurzschlusses, wenn sich im bipolaren Betrieb zwei Elektroden wie beispielsweise die Schneiddrähte oder die großflächigen Elektroden an der Innenseite von Greifarmen berühren. Ein Kurzschluss entsteht beispielsweise dann, wenn es zu einem direkten metallischen Kontakt der beiden Kontaktflächen 2 und 4 kommt und der Strom somit nicht mehr über das Gewebe 9 fließt. Dann findet keine Gewebekoagulation mehr statt.

Figuren 4a und 4b zeigen eine bevorzugte Ausführungsvariante, in der das Instrument mit einer Kurzschlusssicherung 11 versehen ist. Diese Kurzschlusssicherung 11 bewirkt eine mechanische Wegbegrenzung und sorgt dafür, dass im geschlossenen Zustand des Instrumentes ein minimaler Spalt zwischen den beiden Kontaktflächen 2 und 4 vorhanden bleibt und es somit zu keinem direkten metallischen Kontakt beider Kontaktflächen 2 und 4 kommt. Die mechanische Wegbegrenzung kann sowohl unmittelbar neben den Kontaktflächen 2 und 4 angeordnet sein, als auch an irgendeiner anderen Stelle der bewegbaren Schenkel 5 und 6.

Figuren 5a bis 5c zeigen verschiedene Ausführungsvarianten der Anordnung der Kontaktflächen. In Figur 5a sind die Kontaktflächen 2 und 4 auf den distalen Enden der Schenkel 5 und 6 befestigt, wobei die Schenkel aus einem nicht leitendem Material (z. B. PEEK) bestehen. In Figur 5b besteht das Instrument aus leitfähigem Vollmaterial (z. B. chirurgischer Stahl, V2A), wobei bis auf die Kontaktflächen 2 und 4 das Instrument mit einem Isolator (z. B. aus Polyacryl, PA) überzogen ist. In Figur 5c sind die Kontaktflächen 2 und 4 in die distalen Enden der Schenkel 5 und 6 eingepasst, wobei die Schenkel aus einem nicht leitendem Material (z. B. Polyetheretherketone, PEEK) bestehen.

Bei offenen chirurgischen Operationen können durch Gewebeverletzungen körpereigene Flüssigkeiten und Sekrete freigesetzt werden, die sich dann an der zu operierenden Stelle sammeln und damit störend auf den OP-Prozess auswirken. Nach herkömmlicher Methode wird in so einem Fall zusätzlich zu dem eigentlichen chirurgischen Instrument ein Absauginstrument an die zu operierenden Stelle geführt, welches einerseits Platz an der zu operierenden Stelle einnimmt und die OP behindert und andererseits auch separat, ggf. von einer weiteren Person, geführt werden muss. Unter Umständen muss die OP zum Absaugen der Flüssigkeiten sogar unterbrochen werden, da nicht genügend Platz vorhanden ist Absaugung und OP-Instrument gleichzeitig an die OP-Stelle zu bringen.

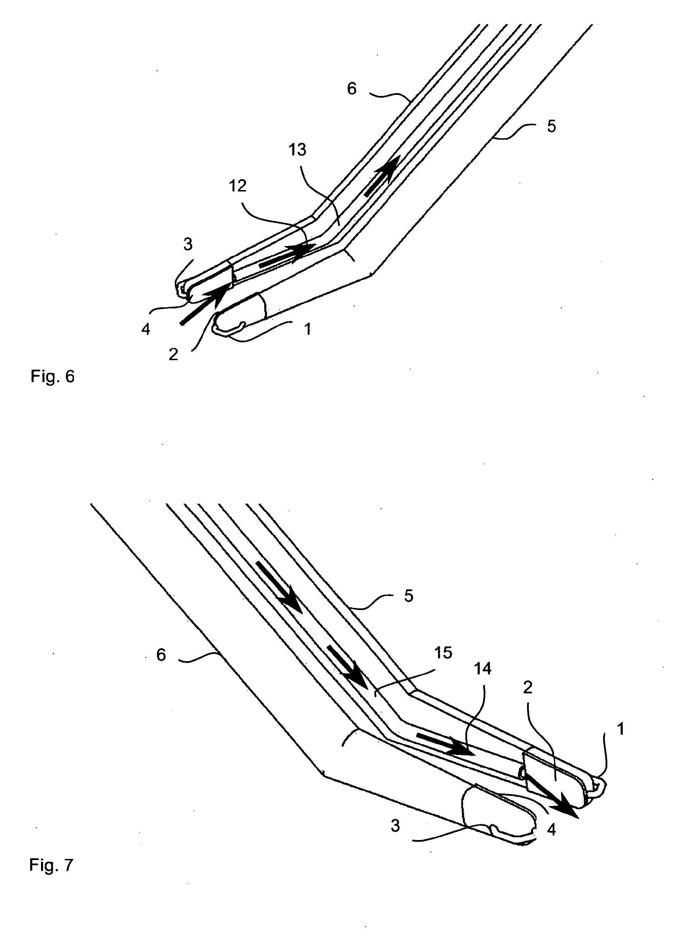

Figur 6 zeigt eine bevorzugte Ausführungsvariante, in der das Instrument mit einem Absaugkanal 13 versehen ist, der ein separates Absauginstrument überflüssig macht. Der Absaugkanal 13 hat eine offene Mündung als Ansaugöffnung im Bereich der distalen Enden der Schenkel 5 und 6, so dass die unerwünschten und störenden Flüssigkeiten direkt im Arbeitsbereich abgesaugt werden. Dies erfolgt parallel zur normalen chirurgischen Arbeit. Der Absaugkanal 13 transportiert die Flüssigkeit 12 ausgehend von der Ansaugöffnung an einem der beiden Schenkel 5 und 6 entlang zu einem offenen proximalen Ende des Ansaugkanals in einem Bereich des Instrumentes, der dem distalen Enden entfernt liegt. An seinem proximalen offenen Ende ist der Absaugkanal 13 mit einem im OP üblichen Absaugsystem zu verbinden.

Bei der Elektrokoagulation kann das Gewebe während der Behandlung austrocknen. Die Folge ist, dass der Übergangswiderstand vom Gewebe zu den elektrochirurgischen Instrumenten als auch der Widerstand des Gewebes selbst mit zunehmender Austrocknung ansteigt und der Prozess nicht mehr einwandfrei funktioniert. In dem Moment muss der Prozess unterbrochen werden, um das Gewebe mit einer ionisierenden, körperverträglichen Flüssigkeit (z. B. sterile physiologische Kochsalzlösung) zu benetzen.

Figur 7 zeigt eine bevorzugte Ausführungsvariante, in der das Instrument mit einem integrierten Befeuchtungssystem versehen ist. Dieses Befeuchtungssystem umfasst einen Spülkanal 15 mit einer offenen Mündung als Auslassöffnung im Bereich der distalen Enden der Schenkel 5 und 6, so dass ionisierende Flüssigkeit 14 direkt in den Arbeitsbereich dosiert werden kann. Dies kann somit parallel zum normalen OP-Prozess erfolgen. Der Spülkanal 15 für die Flüssigkeit 14 verläuft an einem der beiden Schenkel 5 und 6 entlang und ist im Bereich des Instrumentes, der dem distalen Enden entfernt liegt, mit einem im OP üblichem Dosiersystem für derartige Flüssigkeiten zu verbinden.

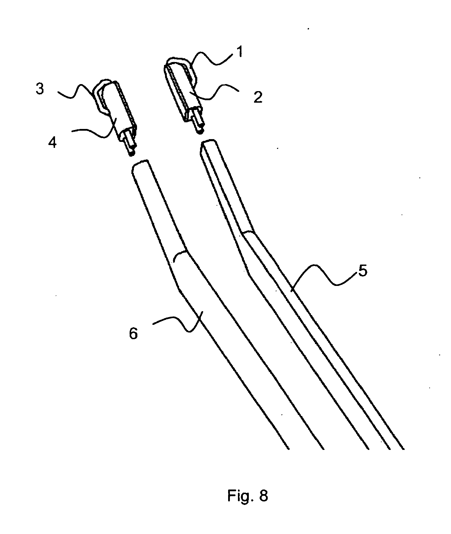

Figur 8 zeigt, dass die distalen Enden der Schenkel 5 und 6, die die Schneiddrähte 1 und 3 und die großflächigen Elektroden 2 und 4 umfassen, austauschbar gestaltet sein können, um so nach Austausch der distalen Enden eine Wiederverwendung des übrigen Instrumentes nach einer Operation zu erlauben.

### Einsatz der Erfindung zur Tonsillektomie:

Speziell bei der Tonsillektomie bietet die Kombination des Hochfrequenzschneidens und des Hochfrequenzkoagulierens in einem Instrument enorme Vorteile. Mittels mehrerer HF-Schnitte durch die Schneiddrähte 1 oder 3 wird die Tonsille langsam aus ihrer Kapsel herausgelöst. Bei diesem Vorgang arbeitet sich der Operateur langsam in die Tiefe vor, um die Tonsille herum, zwischen Tonsille und Tonsillenkapsel. Während des Schneidvorganges wird die Tonsille mit einer Pinzette nach median aus dem Tonsillenbett gezogen. Die Pinzette kann gleichzeitig die Funktion einer Rückleitelektrode besitzen (siehe Fig. 9e). Kleine Blutungen aus Kapillargefäßen werden dabei sofort durch die koagulierende Wirkung des HF-Schnittes auf die Schnittränder gestoppt. Nach dem Herauslösen der Tonsille aus der Kapsel wird die Tonsille am unteren Pol, ebenfalls durch einen HF-Schnitt, abgetragen. Beim Durchtrennen größerer Gefäße, insbesondere Arterien, reicht die koagulierende Wirkung des HF-Schnittes nicht mehr aus, um die Blutung zu stoppen. In diesem Fall wird das Ende des blutenden Gefäßes zwischen die Kontaktflächen 2 und 4 genommen, zusammengedrückt und ein HF-Strom appliziert. Dadurch wird die Öffnung des Gefäßes verschweißt und die Blutung gestoppt. Der Übergang vom Schneiden mit den Kontaktdrähten 1 und 3 zum Koagulieren mit den Kontaktflächen 2 und 4 kann bei der Verwendung der Erfindung ohne Instrumentenwechsel erfolgen. Dadurch können auftretende Blutungen schneller und somit effektiver behandelt werden.

## Patentansprüche

1. Applikationsvorrichtung für das elektrochirurgische Schneiden und Koagulieren von Körpergewebe
welche als ein Greifinstrument mit zwei relativ zueinander beweglichen Greifarmen (5, 6) ausgeführt ist, die jeweils an ihrem distalen Ende je wenigstens zwei Elektroden, nämlich wenigstens eine Koagulationselektrode (2; 4) und wenigstens eine Schneidelektrode (1; 3) aufweisen, **dadurch gekennzeichnet, dass**
wobei die jeweilige Schneidelektrode (1, 3) als ein an der Außenseite eines jeweiligen distalen Endes eines Greifarmes (5; 6) angeordneter Draht ausgebildet ist.

2. Applikationsvorrichtung nach Anspruch 1, **dadurch gekennzeichnet, dass** die Koagulationselektroden (2, 4) als leitende Oberflächen auf einander zugewandten Innenflächen an den distalen Enden der Greifarme (5, 6) ausgeführt sind.

3. Applikationsvorrichtung nach Anspruch 2, **dadurch gekennzeichnet, dass** das Greifinstrument eine mechanische Wegbegrenzung (11) für die Bewegung der Greifarme (5, 6) aufweist, die so ausgebildet ist, dass sie eine gegenseitige Berührung der die Koagulationselektroden bildenden leitenden Oberflächen an den distalen Enden der Greifarme (5, 6) verhindert.

4. Applikationsvorrichtung nach Anspruch 3, **dadurch gekennzeichnet, dass** die mechanische Wegbegrenzung (11) von einem in Richtung des jeweils anderen Greifarms (5; 6) gerichteten Vorsprung auf einer Innenfläche eines Greifarmes (6; 5) gebildet ist.

5. Applikationsvorrichtung nach einem der Ansprüche 1 bis 4, **dadurch gekennzeichnet, dass** wenigstens einer der Greifarme (5, 6) einen Saugkanal (13) mit einer offenen Mündung im Bereich des distalen Endes des Greifarmes (5; 6) aufweist, der an ein Absaugsystem anzuschließen und ausgebildet ist, Flüssigkeit im Bereich des distalen Endes des Greifarmes (5; 6) abzusaugen.

6. Applikationsvorrichtung nach Anspruch 5, **dadurch gekennzeichnet, dass** wenigstens einer der Greifarme (5, 6) einen Spülkanal (15) aufweist, der in einem offenen Ende in der Nähe des distalen Endes des Greifarmes (5; 6) mündet und derart ausgebildet ist, dass er mit seinem proximalen Ende an eine Quelle für Spülflüssigkeit anzuschließen ist und Spülflüssigkeit über seine offene Mündung am distalen Ende austreten kann.

7. Applikationsvorrichtung nach Anspruch 1, **dadurch gekennzeichnet, dass** die Schneidelektroden parallelgeschaltet sind.

8. Applikationsvorrichtung nach Anspruch 7, **gekennzeichnet durch** eine separate Neutralelektrode (17; 18), die mit einem anderen Pol eines Hochfrequenzgenerators (16) zu verbinden ist, als die beiden parallelgeschalteten Schneidelektroden (1, 3).

9. Applikationsvorrichtung nach Anspruch 8, **dadurch gekennzeichnet, dass** die separate Neutralelektrode als elektrochirurgische Pinzette (18) ausgebildet ist.

## Claims

1. Application device for the electrosurgical cutting and coagulation of body tissue, which is in the form of a gripping instrument with two gripping arms (5, 6) which are movable relative to each other, wherein the distal ends of the gripping arms (5, 6) respectively have at least two electrodes, namely at least one coagulation electrode (2; 4) and at least one cutting electrode (1; 3), **characterised in that** the respective cutting electrode (1, 3) is designed as a wire arranged on the outside of a respective distal end of a gripping arm (5; 6).

2. Application device according to 1, **characterised in that** the coagulation electrodes (2, 4) are in the form of conducting surfaces on mutually facing inside faces on the distal ends of the gripping arms (5, 6).

3. Application device according to claim 2, **characterised in that** the gripping instrument has a mechanical travel limiting means (11) for the movement of the gripping arms (5, 6), which is designed so that it prevents mutual contacting of the conducting surfaces forming the coagulation electrodes at the distal ends of the gripping arms (5, 6).

4. Application device according to claim 3, **characterised in that** the mechanical travel limiting means (11) is formed by a projection on an inside face of a gripping arm (6; 5), the projection being directed in the direction of the respective other gripping arm (5; 6).

5. Application device according to any one of claims 1 to 4, **characterised in that** at least one of the gripping arms (5, 6) has a suction passage (13) with an open mouth opening in the region of the distal end of the gripping arm (5; 6), which is to be connected to a suction removal system and is adapted to suction fluid in the region of the distal end of the gripping arm (5; 6).

6. Application device according to claim 5, **characterised in that** at least one of the gripping arms (5, 6) comprises a flushing passage (15), which opens in an open end in the proximity of the distal end of the gripping arm (5; 6) and is designed so that it is connected with its proximal end to a source for flushing fluid and flushing fluid can exit via its open mouth at the distal end.

7. Application device according to claim 1, **characterised in that** the cutting electrodes are connected in parallel.

8. Application device according to claim 7, **characterised by** a neutral electrode (17; 18), which is to be connected to a different pole of a high frequency generator (16), which is separate from the two parallel-connected cutting electrodes (1, 3).

9. Application device according to claim 8, **characterised in that** the separate neutral electrode is designed in the form of electrosurgical tweezers (18).

## Revendications

1. Dispositif d'application pour la découpe électrochirurgicale et la coagulation de tissu corporel, lequel dispositif est réalisé sous la forme d'un instrument de préhension comportant deux bras de préhension (5, 6) qui sont mobiles l'un par rapport à l'autre et qui comportent chacun à leur extrémité distale au moins deux électrodes, à savoir au moins une électrode de coagulation (2 ; 4) et au moins une électrode de découpe (1 ; 3),
**caractérisé en ce que** l'électrode de découpe respective (1, 3) est conformée en fil disposé du côté extérieur d'une extrémité distale respective d'un bras de préhension (5 ; 6).

2. Dispositif d'application selon la revendication 1, **caractérisé en ce que** les électrodes de coagulation (2, 4) sont réalisées sous la forme de surfaces conductrices, dont les faces internes sont dirigées l'une vers l'autre, au niveau des extrémités distales des bras de préhension (5, 6).

3. Dispositif d'application selon la revendication 2, **caractérisé en ce que** l'instrument de préhension présente une limitation de course mécanique (11), destinée à limiter le déplacement des bras de préhension (5, 6), qui est conformée de façon à empêcher les surfaces conductrices, formant les électrodes de coagulation, de venir en contact l'une avec l'autre au niveau des extrémités distales des bras de préhension (5, 6).

4. Dispositif d'application selon la revendication 3, **caractérisé en ce que** la limitation de course mécanique (11) est formée par une saillie, orientée en direction de l'autre bras de préhension (5 ; 6), sur une face intérieure d'un bras de préhension (6 ; 5).

5. Dispositif d'application selon l'une des revendications 1 à 4, **caractérisé en ce que** au moins un des bras de préhension (5, 6) possède un canal d'aspiration (15) présentant une embouchure ouverte dans la région de l'extrémité distale du bras de préhension (5 ; 6) qui peut être raccordée à un système d'aspiration et qui est conformée pour aspirer un liquide dans la région de l'extrémité distale du bras de préhension (5 ; 6).

6. Dispositif d'application selon la revendication 5, **caractérisé en ce que** au moins un des bras de préhension (5, 6) possède un canal de lavage (15) qui débouche dans une extrémité ouverte à proximité de l'extrémité distale du bras de préhension (5 ; 6) et qui est conformé de façon à pouvoir être raccordé, à son extrémité proximale, à une source de liquide de lavage et de façon à ce que le liquide de lavage puisse sortir par son embouchure ouverte à l'extrémité distale.

7. Dispositif d'application selon la revendication 1, **caractérisé en ce que** les électrodes de découpe sont montées en parallèle.

8. Dispositif d'application selon la revendication 7, **caractérisé par** une électrode neutre séparée (17 ; 18) qui peut être reliée à un pôle d'un générateur à hautes fréquences (16) différent du pôle des deux électrodes de découpe (1, 3) montées en parallèle.

9. Dispositif d'application selon la revendication 8, **caractérisé en ce que** l'électrode neutre séparée est conformée en pinces électrochirurgicales (18).
